Europäisches Patentamt

**European Patent Office**

**Office européen des brevets**

(19)

(11) Publication number: **0 039 163**
**A1**

## (12) EUROPEAN PATENT APPLICATION

(21) Application number: **81301604.5**

(22) Date of filing: **10.04.81**

(51) Int. Cl.³: **A 01 G 7/04**
**A 61 N 1/42**

(30) Priority: **17.04.80 US 140443**

(43) Date of publication of application:
**04.11.81 Bulletin 81/44**

(84) Designated Contracting States:
**BE DE FR GB NL**

(71) Applicant: ELECTRO-BIOLOGY, INC
300 Fairfield Road
Fairfield New Jersey 07006(US)

(72) Inventor: Pilla, Arthur A.
371 Martom Road
Hyckoff New Jersey(US)

(74) Representative: Wright, Peter David John et al,
R.G.C. Jenkins & Co. 53-64 Chancery Lane
London WC2A 1QU(GB)

(54) Method and means for electromagnetic stimulation of a vegetative process.

(57) The invention contemplates method and apparatus for altering the growth behavior of living plant cells by inducing voltage and concommitant current pulses of specific time-frequency-amplitude relations therewithin.

./...

FIG. I.

TO SOURCE OF
EXCITATION

FIG. 2.

METHOD AND MEANS FOR ELECTROMAGNETIC
STIMULATION OF A VEGETATIVE PROCESS

Background of the Invention

This invention relates to the treatment of living plant cells by altering their interaction with charged species in their environment. In particular, the invention relates to a controlled modification of plant-cell growth behavior by the application of encoded electrical information. More particularly, the invention provides for the intra-cellular application, via noncontacting direct inductive coupling, of one or more electrical voltage and concommitant current signals conforming to a highly specific pattern.

Several attempts have been made in the past to elicit a response of living cells and/or tissue to electrical signals. Much of this work is reviewed and discussed in Ryaby, et al. Patent No. 4,105,017, particularly insofar as such responses have been elicited for human and other animal-cell behavior. Reference is made to said patent for extensive background discussion.

I have discovered that certain highly beneficial modifications of otherwise normal plant-cell metabolism may be specifically and selectively induced via electro-

magnetic coupling using apparatus and techniques having much in common with that disclosed in said patent and in pending Ryaby, et al. application Serial No. 887,485, filed March 17, 1978.

Brief Statement of the Invention

It is an overall object of the invention to provide a method and means for selectively eliciting, via electromagnetic coupling, a specifically modified plant-cell response.

It is another object to achieve the foregoing object in specific application to selectively accelerated seed-germination.

Another object is to achieve said overall object in specific application to plant-root development.

A further object is to achieve said overall object in specific application to selective retardation or stopping of normal plant-cell development.

It is a still further object to achieve said overall object in specific application to accelerated meristem culture development.

A further overall object is to provide method and means for evoking the non-linear electrical response of a plant cell in selective enhancement or diminution of the characteristic electrochemical behavior of the cell.

-3-

The foregoing objects and various further features of novelty are achieved by the invention, wherein growth behavior of living plant cells is selectively altered by electromagnetically inducing low-level voltage and concommitant current pulses of specific time-frequency-amplitude relations therewith. More particularly, by controlled application of particular excitation levels and repetition rates of such pulses, it is found that such normal plant-development processes as seed-germination, root-development and ripening may be selectively accelerated or retarded.

Detailed Description

The invention will be illustratively described in detail in conjunction with the accompanying drawings, in which:

Fig. 1 is a simplified view in perspective, to show electromagnetic apparatus of the invention, operative upon specimen plant cells in a removable container;

Fig. 2 is a vertical sectional view of the removable container of Fig. 1, specifically fitted for accelerated germination and initial root development of specimen plant seeds;

Fig. 3 is an electrical block diagram schematically showing means for exciting the apparatus of Fig. 1;

-4-

Fig. 4 is a waveform diagram to illustrate operation of the apparatus when thus excited;

Fig. 5 is a diagram to illuminate discussion of dual-coil placement considerations; and

Figs. 6 to 9 are simplified vertical sectional diagrams to illustrate various apparatus modifications and different plant-biology applications of the invention.

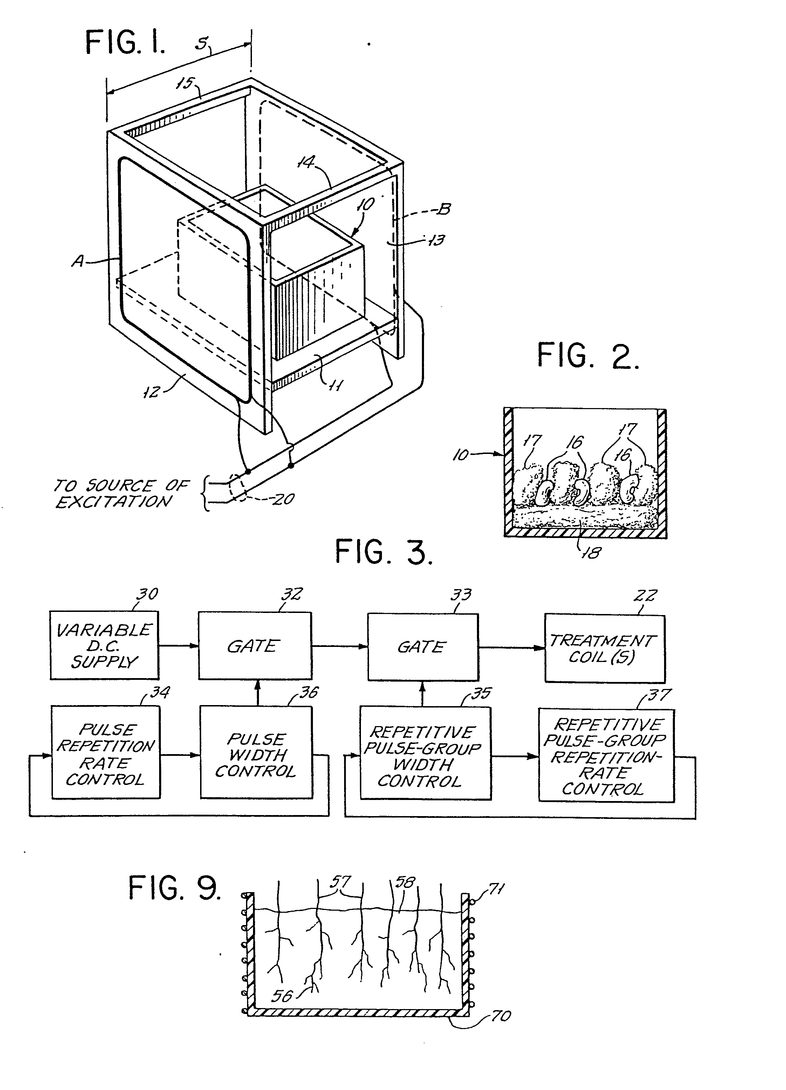

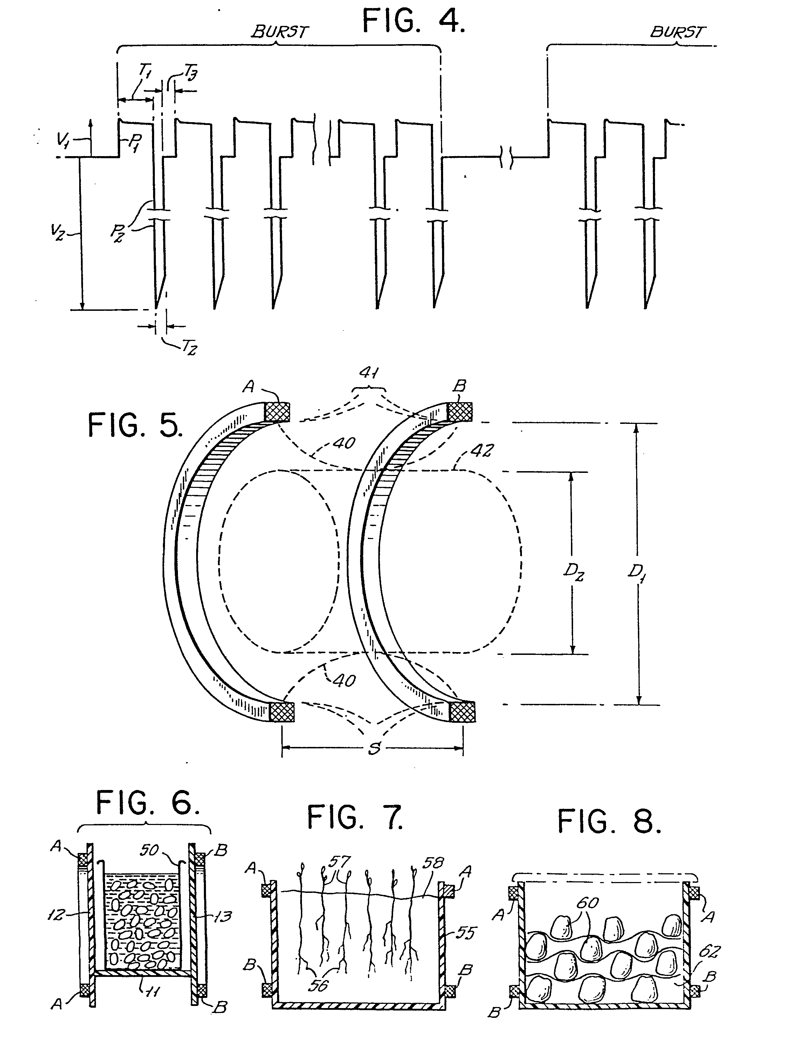

In Fig. 1, plant seed to be subjected to the method of the invention is suitably supported within an open box-like container 10 of non-paramagnetic material, and the container is removably positionable upon a supporting shelf 11 which extends between two parallel upstanding sidewalls 12-13. Spacer bars 14-15 connect corresponding upper corners of the sidewalls 12-13 to assure their parallel relation. The sidewalls 12-13 are generally square, and each of them mounts an electrical coil A-B which may be preformed and then assembled, as by means of suitable adhesive or ties, to the outer exposed face of the associated wall 12-13; and the entire coil-mounting structure 11-12-13-14-15 is also of non-paramagnetic material. The coils A-B are duplicates of each other and are interconnected in flux-aiding relation, when excited by a suitable source; as shown, they are electrically connected in parallel. The seed-supporting volume of container 10 is preferably sized and positioned on shelf 11 so as to enclose all cells to be treated, within the geometrical volume defined by and between the coils A-B; also, the spacing S between coils A-B is preferably less than the effective diameter of the respective coils, as will later be explained in connection with Fig. 5. The particular coils A-B in Fig. 1 are square and therefore their effective diameter may be approximated by the peripheral extent of the square, divided by $\pi$.

Fig. 2 shows illustrative seed-retaining detail, within the container 10. Each of plural seeds 16 is supported in a moist environment, by and between spaced cotton tufts 17, over a bed 18 of moisture-retaining material such as cotton, the cotton being wetted to assure the moist environment for each of the seeds 16.

Referring to Fig. 3, a variable d-c supply 30 is coupled through gate means 32 to the treatment coil 22 (or coils, as the case may be and as will more fully appear below); in conjunction with the apparatus of Fig. 1, the coil(s) 22 of Fig. 3 will be understood to symbolize connection to the line 20 which serves coils A and B. The gate 32 is under the control of control units 34-36, which cause a pulse signal consisting of repetitive pulses of electrical potential to be applied to the coil means 22. Each pulse cycle, as shown in

Fig. 4, is composed of a pulse portion $P_1$ of first polarity followed by a pulse portion $P_2$ of opposite polarity. The control units 34-36 may typically be monostable multivibrators, e.g., to generate appropriate timing signals, and they may be variable to control pulse duration and repetition rate within desired limits, while the use of a variable d-c supply 30 permits variation of the pulse-signal amplitude as desired.

With the pulse-generator apparatus thus far described, the output signal for coil excitation is in what has been described as Mode 1, being a steady succession of asymmetrical voltage pulses, the asymmetry being attributable to different time constants and being characterised first pulses $P_1$ of lesser amplitude $V_1$ and greater duration $T_1$, as compared with second pulses $P_2$ of greater amplitude $V_2$ and lesser duration $T_2$, there being a dwell $T_3$ between successive pulse cycles, depending upon circuit constants. The Mode 1 signal will produce cell-modulating effects, but I have thus far found a pulse-train or Mode 2 excitation signal to be preferred for plant-cell stimulation.

When pulse-train operation (Mode 2) is employed, additional timing circuitry similar to units 34 and 36 is employed to define the burst-segment duration and the burst segment repetition rate. As shown, control units 35-37 control gate 33 to produce a coil-excitation signal of the described asymmetrical nature, but wherein the control units 35-37 determine the number of pulses in a burst and the time between successive bursts of pulses.

It has been found that the signal across the treatment coil or coils, and hence the induced signal within plant cells under treatment, should satisfy certain criteria. These criteria will be specified with respect to the signal as induced in the cells under treatment. Such induced signal may be monitored, if desired, by use of an auxiliary monitoring probe or pickup coil (not shown) which is positioned at a distance from the coils A-B corresponding to the distance of the plant cells under treatment from such coils, as is explained in detail in said patent. It suffices here to state that a typical pickup coil that has been employed is circular, about one-half centimeter in diameter, and with about 67 or 68 turns of small gauge enamel-coated wire. The potential developed by the coil is divided by the length of the wire (in centimeters) to provide an induced voltage per centimeter number that is closely related to the volts per centimeter induced in the plant cells under treatment.

EXAMPLE

In order to demonstrate efficacy in application to plant cells, five identical structures as described in connection with Fig. 1 were identically prepared as discussed in connection with Fig. 2, using a batch of six beans in each of the containers 10. The beans were a commercially available hybrid variety of bush-type green beans, known as "tendercrop". Care was taken that each bean was individually supported in substantially the same moisture environment, involving tap-water wetting of the supporting cotton elements 17-18. Each of the coils A-B was of generally square configuration, 4 in. by 4 in., to define a 16-in. perimeter, having an effective diameter of about 5 inches, being defined by 60 turns of No. 16 enamel-coated copper wire; and the spacing S between coils was 2.5 inches. Coil excitation with the circuit of Fig. 3 provided a first-pulse amplitude $V_1$ of 15 mv and duration $T_1$ of 200 μsec., and a second-pulse amplitude $V_2$ of 180 mv and duration $T_2$ of 12μsec., with a short dwell $T_3$ of 8μsec. between pulses. Bursts of five milliseconds were administered concurrently to each batch, at different burst-repetition rates, and there was no other variable. The ambient temperature within each batch remained substantially constant at $20^{\circ}$C. $\pm$ $0.5^{\circ}$C., and the treatment continued for five days and nights, with all batches receiving the same quantum of normal indoor sunlight exposure. A control sample batch of beans was supported in an identical moist-cotton environment in a sixth container 10 which was not exposed to the time-varying magnetic-flux environment applicable to the five like Fig. 1 systems. At the end of the five days, the beams in containers 10 were inspected for signs of germination and root development, with the following results:

| Batch | Burst Duration | Burst-Repetition Rate | Root Length (mean) |
|-------|----------------|-----------------------|--------------------|
| 1 | 5 ms | 5 kHz | 0 |
| 2 | 5 ms | 10 kHz | 0.5 inch |
| 3 | 5 ms | 15 kHz | 1.5 inch |
| 4 | 5 ms | 20 kHz | 1.5 inch |
| 5 | 5 ms | 25 kHz | 3/4 inch |
| 6 | None | Not applicable | 0 |

In all cases, except for the control sample of batch 6, the probe-coil measurement at the region of seed immersion in the applicable magnetic field, indicated and confirmed the above-noted values of $V_1$, $V_2$, $T_1$, $T_2$ and $T_3$ within all bursts. And a platinum-wire resistance element applied to one bean of each batch failed to indicate any appreciable rise in temperature throughout the five-day exposure to the moist and time-varying magnetic environments; the platinum element had the capability of measuring a $0.01^\circ C$ rise in temperature, if

-10-

such rise had occurred.

It is concluded from the foregoing example that a definite "window" exists for favorable and effective acceleration of the germination and root-development phases of the growth cycle of the indicated plant cells. And from basic similarity between all varieties of plant cells, an analogous conclusion is justified (a) for corresponding phases of the growth cycle of other plant-cell varieties and (b) for other phases of the growth cycle for all plant-cell varieties. In all cases, it is considered important for most favorable and uniformly predictable results that magnetic-flux distribution be substantially uniform and uniformly oriented (substantially parallel to the coil axis) in the region of plant-cell exposure and treatment. The latter point will be briefly discussed in connection with the pair of circular coils A-B of Fig. 5.

On an elemental basis, it is convenient to consider the circular-coil situation depicted in Fig. 5, wherein like circular coils A-B of inside diameter $D_1$ are positioned on a common central axis of symmetry, at parallel planes which are spaced apart by the distance S, and wherein the coils A-B are excited in flux-aiding relation. If the spacing S is sufficiently small in relation to the diameter $D_1$, then substantially all flux

0039163

-11-

lines within coils A-B will extend continuously therebetween, on a generally straight alignment which may even neck down, as suggested by the profile 40. If the spacing S is greater (again in relation to the diameter $D_1$), some stray-flux lines 41 will develop, to the detriment of the development of uniform high-density flux in the central span S. Generally, in view of the necking down (40), and in view of the treatment zone being generally at the center of span S, it is convenient to consider the coils A-B as being desirably effective in producing the uniform flux distribution over an imaginary cylinder 42 of diameter $D_2$, tangent to the neck-down profile 40. From experience to date, it can be stated that for plant-cell application of the character presently described, the span S should be equal to or less than the diameter $D_1$, and of course $D_2$ (the effective diameter of the zone of plant-cell treatment) will always be considerably less than $D_1$, being substantially equal to $D_1$ only when coils A-B are closely adjacent. As a practical consideration in the application of dual coils to a plant-cell specimen or batch, it is believed that the nominal inside diameter $D_1$ of the coils should be at least 1.5 times the diameter $D_2$ of the effective cell-treatment zone, and this has been found to be a reliable approach for coil spacing S substantially equal

-12-

to the inside diameter $D_1$.

Further applications, methods and structures of the invention will be indicated in discussion of the successive embodiments of Figs. 6 to 9.

In Fig. 6, a container 50 of non-paramagnetic material is filled with plant seeds for which the germination phase is to be accelerated; for example, a normal germination period of about three weeks is to be reduced to a few days. The seeds in container 50 are covered with water while, say, one of the burst-repetition rates (for one of samples 2 to 5 given above) characterizes the 5 msec. bursts of pulses delivered via flux-aiding coils A-B. After such exposure, the seeds are well along in their germination phase and may be set out for planting. It will be seen that by making possible such accelerated germination, the plant farmer need not suffer from a spring season subject to late cold spells. He can await the arrival of assuredly good weather, accelerate germination through the moist and pulse-characterized varying magnetic field, and then plant without fear of having lost an important part of the growing season.

Fig. 6 also illustrates application of the invention to acceleration of a meristem culture development, applicable for example to propagation of certain plants such as orchids. In that event, the container 50 may suitably be a flask containing comminuted plant buds in a liquid nutrient medium, consisting illustratively of coconut milk (for hormones), fish emulsion (for fertilizer), and a saline solution of roughly the salinity of plant tissues. The development of such comminuted buds into as many discrete plants is greatly accelerated by the exposure of the thus-laden flask 50 to the time-varying magnetic field established by and between coils A-B.

In the arrangement of Fig. 7, horizontal coils A and B each encircle a non-paramagnetic pot or vessel 55, at a vertical spacing between coils that is less than the coil diameter, thus establishing vertical orientation of magnetic lines in a substantially uniform high-density flux distribution between the coils. The arrangement is favorably suited to the rapid development of root structure 56 from plant cuttings 57 which have been planted in a moist suitable rooting medium 58, such as potting soil, vermiculite, or sand; illustrative plants applicable to such stimulation of potted cuttings include african violets, gardenias, and species of ficus including rubber plants. Again, a process normally requiring several weeks is reducible to several days,

-14-

upon application of specific excitation signal patterns to the coils A-B, connected in flux-aiding relation.

The arrangement of Fig. 8 will be recognized for its similarity to Fig. 7, the difference being one of scale, in that pieces of fruit 60 are stored within the non-paramagnetic container 61, in preferably stacked and spaced array as determined by separation sheets 62. If the fruit 60 was picked short of full ripeness, then application of suitable low-level time-varying excitation signals to coils A-B will create a cell-metabolism enhancing environment, to enable accelerated ripening to completion, for such quantities of fruit as may be presently needed in quickly ripened condition. If on the other hand, the fruit 60 is already ripe and is being stored for postponed use, then preferably air within container 61 is displaced by an atmosphere of nitrogen, and the protective atmosphere is retained by suitable cover means 63; at the same time a regimen of excitation-signal amplitudes and patterns is established by adjustments within the circuit of Fig. 3, whereby cells within the fruit 60 are subjected to metabolism-inhibiting pulses. In other words, for plant-cell storage and preservation purposes, one selects excitation-pulse levels which are high enough

to substantially frustrate and prevent the normal metabolism phase associated with over-ripening.

The arrangement of Fig. 9 will be recognized for its similarity to Fig. 7, in that plant cuttings have been potted in moist medium 58, but the non-paramagnetic pot or container 70 mounts but a single multi-turn coil 71 which circumferentially envelops the vertical-wall structure, for the full vertical extent of container 70. It goes without saying that such a single coil 71 amply provides the capability of establishing high-density uniform flux distribution within the potted volume containing the cuttings 57 and that, therefore, magnetically induced stimulation of the cuttings for root development may be as effective as in the case of Fig. 7.

A full and complete understanding of the mechanism by which plant-cell growth is modified by the invention has yet to be given. It is presently believed, however, that each plant cell importantly includes an outer layer which in the indicated moist environment is relatively conductive, thus establishing for each cell what may be viewed as a shorted single-turn secondary, inherently susceptible to voltage induction and therefore voltage generation within each cell that is exposed to the time-varying magnetic field established by the particular

-16-

excited treatment coil or coils. It is believed that each cell exhibits non-linear electrical response to cell-induced voltage in the presence of a low-level time-varying magnetic field, the net electrochemical response of each cell during each cycle of a time-varying signal being given by:

$$\Delta P = \frac{1}{T_1} \int_0^{T_1} (1 - e^{\frac{-t}{TM_1}}) \, dt - \frac{1}{T_2} \int_0^{T_2} (1 - e^{\frac{-t}{TM_2}}) \, dt,$$

where $\Delta P$ is the net perturbation for the basic waveform of said signal, where said signal has opposed successive polarity segments of duration $T_1$ and $T_2$, respectively, and where $TM_1$ and $TM_2$ are the respective forward and reverse time constants of said cell. Thus, non-linearity in the response of the cell itself may perhaps be as much responsible as any other factor in achieving the described results.

For a low-level time-varying electrical signal of arbitrary waveform to achieve a net biological effect, it is believed that the receiver (the plant cell) must possess non-linear characteristics. For example, if a net perturbation (i.e., net, over a period of time) of the amount of a specifically adsorbed (bound) ion at a cell-membrane site is the target or goal, then the use of induced current can cause this to occur only if the process exhibits a certain degree of irreversability (i.e., non-linearity); in the case of plant-cell stimulation, it is believed that activation of calcium-binding protein may be specifically involved; in addition, it appears that adsorption and/or transmembrane passage of cations (e.g., $Ca^{++}$, $Na^+$, $K^+$) are also believed to be significant to the response. This non-linear property is also coupled with the basic kinetics (relaxation time) of the process. In other words, the parameters of the perturbing signal must be such that efficient coupling to the biological cell process can be achieved without such excessive amplitude as would remove the signal from the low-level category and would invite the occurrence of other non-electrical effects, such as thermal effects, which it will be recalled from the present specific example are not measurably present for the low-level amplitude example given.

Quantitative assessment of the manner by which informational signals coupled to cellular processes having the just-described characteristics can be achieved by considering that (i) a surface process is involved, (ii) its basic kinetics are exponential in nature, (iii) net response during each cycle of a signal of arbitrary waveshape is given by the above-indicated expression for $\Delta P$, the net perturbation occurring for the duration of the waveform (i.e., $T_1 + T_2$), (iv) the various repetitive natures of the signal burst and/or repetition rate, as well as the basic waveform-timing characteristics can be conveniently quantitated via spectral analysis using Laplace transformation, and (v) the overall net perturbation $\overline{\Delta P}$ can then be described in terms of its frequency content in the context of the electrical waveform characteristics.

In the above manner, the desirable characteristics of a waveform of any shape can be chosen to provide a correlation between its frequency spectrum (as determined by the electrochemical cell-surface process) and the elicited biological response. Generally, there will be an amplitude range (or ranges) over which the system receives an adequate "dose"-to-modulate function. The number of specific ranges depends upon system complexity. In other words, the more biological stages having distinct control processes, the more amplitude ranges may be present for selective operation upon the different control processes.

The described invention will be seen to have achieved the stated objects. In the aspect of plant-cell germination and root development, the invention is found to achieve truly striking results with important and beneficial economic and environmental-impact implications.

While the invention has been described in detail for the specific illustrative apparatus and example, it will be understood that modifications may be made without departing from the scope of the invention.

CLAIMS:

1. A method of altering the growth behavior of living plant cells, said method comprising subjecting said cells to a moist environment wherein a plurality of individual cells each exhibit non-linear electrical response to cell-induced voltage in the presence of a low-level time-varying magnetic field; the net electrochemical response of each of said individual cells during each cycle of a time-varying signal being given by:

$$\Delta P = \frac{1}{T_1} \int_0^{T_1} (1 - e^{\frac{-t}{TM_1}}) \, dt - \frac{1}{T_2} \int_0^{T_2} (1 - e^{\frac{-t}{TM_2}}) \, dt,$$

where $\Delta P$ is the net perturbation for the basic waveform of said signal, where said signal has opposed successive polarity segments of duration $T_1$ and $T_2$ respectively, and where $TM_1$ and $TM_2$ are the respective forward and reverse time constants of each said individual cell; and additionally subjecting said individual cells to the electromagnetic induction of electrochemical time-varying informational signals which controllably modify a fundamental process involved in vegetative growth behavior of said cells when applied in predetermined time and informational sequence, the successive polarity segments of said signal being at predetermined amplitude and for predetermined duration of repetitive cycling to effect an elicited biological response of said cells.

2. The method of claim 1, wherein said cells are component elements of a plant seed capable of ultimate germination in a moist environment.

3. The method of claim 1, wherein said time-varying signal is asymmetrical with a first polarity pulse segment of greater duration and lesser amplitude as compared with a second polarity pulse segment of lesser duration and greater amplitude.

4. The method of claim 1, wherein said cells are component elements of a plant cutting capable of root development when a portion of said cutting is supported in a moist nutritional environment.

5. The method of claim 1, wherein said cells are component elements of each of a plurality of plant seeds capable of germination in a moist environment, the moist environment being provided by water-immersion of said seeds throughout the period in which said cells are subjected to the electromagnetic induction of said informational signals.

6. The method of claim 4, wherein the plant cutting is one of a plurality of similar cuttings similarly exposed to the same moist environment and to the same electromagnetic-induction signals.

7. The method of claim 1, wherein said cells are component elements of each of a plurality of comminutions of a plant bud capable of meristem culture, said comminutions being immersed in a nutrient-solution environment.

8. The method of claim 7, wherein said nutrient environment is a saline solution which includes hormone and fertilizer materials as essential components.

9. The method of claim 1, wherein said cells are component elements of ripened fruit, and wherein the informational signals to which said fruit is subjected are at an amplitude level above that at which said cells respond with ripening metabolism.

10. The method of claim 1, wherein said cells are component elements of unripened fruit and wherein the informational signals to which said fruit is subjected are at an amplitude level at which said cells respond with ripening metabolism.

11. The method of claim 1, in which time-varying signals characterize a substantially uniform directionally aligned magnetic-field environment with a confined geometrical volume which encloses said plant cells.

12. The method of claim 11, in which said directionally aligned magnetic-field environment is provided by a single multi-turn coil of length and internal peripheral extent to establish said volume.

13. The method of claim 11, in which said directionally aligned magnetic-field environment is provided by two like multi-turn coils in axially spaced and axially aligned array, the axial spacing being less than the effective internal diameter of said coils, and said coils being excited in flux-aiding relation.

14. An electromagnetic treatment device for modification of living plant cells by a specific and selective change in electrical environment, comprising magnetic-circuit means including two axially spaced electrical coils connected for excitation in flux-aiding relation, retaining means adapted to fixedly hold said coils at said spacing to thereby define a treatment zone between said coils, whereby upon excitation said coils establish a flux-development axis through said zone, support means of non-paramagnetic material adapted to support living plant cells and to provide a moist environment of such supported cells within said treatment zone, and electric-circuit means for exciting said magnetic-circuit means with a repetitive low-voltage signal cycle designed for such electromagnetic coupling to the plant cells as to induce a specific non-linear electrochemical cell response.

15. An electrochemical treatment device for modification of living plant cells by a specific and selective change in electrical environment, comprising magnetic-circuit means including a single multi-turn electrical coil extending longitudinally along a central flux-development axis and surrounding and defining a geometric volume within and for such longitudinal extent, support means of non-paramagnetic material adapted to support living plant cells and to provide a moist environment of such supported cells within said volume, and electric-circuit means for exciting said magnetic-circuit means with a repetitive low-voltage signal cycle designed for such electro-magnetic coupling to the plant cells as to induce a specific non-linear electrochemical cell response.

16. An electromagnetic treatment device for modification of living plant cells by a specific and selective change in electrical environment, comprising magnetic-circuit means including two spaced elements and retaining means adapted to hold said elements in fixedly spaced relation on opposite sides of a treatment zone, said elements being adapted to establish a flux-development axis therebetween and through said zone, support means of non-paramagnetic material adapted to support living plant cells and to provide a moist environment of such supported cells within said treatment zone, and electric-circuit means for exciting said magnetic-circuit means with a repetitive low-voltage signal cycle designed for such electromagnetic coupling to the plant cells as to induce a specific non-linear electrochemical cell response.

17. An electromagnetic treatment device comprising an upwardly open container of non-paramagnetic material, electrical-coil means including a multi-turn winding peripherally carried by said container and having an upstanding flux-development axis through the volume of said container, said container being adapted to support living plant cells therewithin and to provide a moist environment for such supported cells within the included volume of said electrical-coil means, and electric-circuit means for exciting said magnetic-circuit means with a repetitive signal cycle designed for such electro-magnetic coupling to the plant cells as to induce a specific non-linear electrochemical cell response.

18. The treatment device of claim 17, wherein said electrical-coil means is a single coil helically developed around said container and of substantially the vertical extent of said container.

-8-

19. The treatment device of claim 17, wherein said electrical-coil means comprises two like multi-turn coils at vertically spaced locations within the vertical extent of said container, said coils being connected in flux-aiding relation and the spacing between said coils being less than the effective diameter of said coils.

20. The method of claim 1, wherein the repetitive cycling is for a limited burst period and wherein the burst is repeated at a burst-repetition rate to effect an elicited biological response of said cells.

21. A method of altering the growth behavior of living plant cells, said method comprising subjecting said cells to a moist environment wherein a plurality of individual cells each exhibit a non-linear electrochemical response to cell-induced voltage in the presence of a time-varying magnetic field, and subjecting said individual cells to the electromagnetic induction of electrochemical time-varying informational signals which controllably modify a fundamental process involved in vegetative growth behavior of said cells.

22. The method of claim 21, in which said informational signals are at a level selected above that at which the normal growth behavior of said cells is enhanced.

23. The method of claim 21, in which said informational signals are at a level selected sufficiently low as to enhance the normal growth behavior of said cells.

24. The method of claim 21, in which said informational signals are at a level selected sufficiently high as to substantially diminish the normal growth behavior of said cells.

FIG. I.

TO SOURCE OF
EXCITATION

FIG. 2.

FIG. 3.

| VARIABLE D.C. SUPPLY | → | GATE | → | GATE | → | TREATMENT COIL (S) |
|---|---|---|---|---|---|---|

| PULSE REPETITION RATE CONTROL | → | PULSE WIDTH CONTROL | | REPETITIVE PULSE-GROUP WIDTH CONTROL | → | REPETITIVE PULSE-GROUP REPETITION-RATE CONTROL |
|---|---|---|---|---|---|---|

FIG. 9.

2

0039163

# FIG. 4.

# FIG. 5.

# FIG. 6.

# FIG. 7.

# FIG. 8.

European Patent Office

**EUROPEAN SEARCH REPORT**

Application number

EP 81 30 1604.5

| | DOCUMENTS CONSIDERED TO·BE RELEVANT | | CLASSIFICATION OF THE APPLICATION (Int. Cl.³) |
|---|---|---|---|
| Category | Citation of document with indication, where appropriate, of relevant passages | Relevant to claim | |
| | FR – A1 – 2 369 850 (J. FRAGNET)<br>* claims 1 to 5; fig. 10 *<br>— | 1,3 | A 01 G    7/04<br>A 61 N    1/42 |
| | US – A– 2 308 204 (W.P. BERKELEY)<br>* entire document *<br>— | 1 | |
| D | US – A – 4 105 017 (J.P. RYABY et al.)<br>* claim 1; fig. 5b *<br>— | 1,3 | |
| P | DE – A1 – 2 841 933 (KABEL– UND METALL–WERKE GUTEHOFFNUNGSHÜTTE AG)<br>* page 5, line 15 to page 7, line 16 *<br>— | 1,8 | **TECHNICAL FIELDS SEARCHED** (Int. Cl.³)<br><br>A 01 C    1/00<br>A 01 G    7/00<br>A 61 N    1/40<br>A 61 N    1/42 |
| A | US – A – 3 893 462 (M.R. MANNING)<br>* abstract;·fig. 3, 4 *<br>— | 1 | |
| A | EP – A1 – 0 005 713 (E. RASCHE et al.)<br>— | | |
| A | DE – B – 1 302 383 (F.K. KÖNIG)<br>———— | | |

CATEGORY OF CITED DOCUMENTS

X: particularly relevant
A: technological background
O: non-written disclosure  .
P: intermediate document
T: theory or principle underlying the invention
E: conflicting application
D: document cited in the application
L: citation for other reasons

&: member of the same patent family, corresponding document

| | The present search report has been drawn up for all claims | |
|---|---|---|
| Place of search | Date of completion of the search | Examiner |
| Berlin | 03–07–1981 | SCHOFER |

EPO Form 1503.1   06.78